(19) Europäisches Patentamt
European Patent Office
Office européen des brevets

(11) Numéro de publication: **0 195 734**
**A2**

(12) # DEMANDE DE BREVET EUROPEEN

(21) Numéro de dépôt: 86420080.3

(22) Date de dépôt: 20.03.86

(51) Int. Cl.⁴: C 07 F 9/38
C 07 F 9/40, C 07 D 265/16

(30) Priorité: 21.03.85 FR 8504433

(43) Date de publication de la demande:
24.09.86 Bulletin 86/39

(84) Etats contractants désignés:
AT BE CH DE FR GB IT LI LU NL SE

(71) Demandeur: RHONE-POULENC AGROCHIMIE
14-20, rue Pierre Baizet
F-69009 Lyon(FR)

(72) Inventeur: Corbet, Jean-Pierre
Le Marronnier Domaine de Charrière Blanche
F-69130 Ecully(FR)

(74) Mandataire: Brachotte, Charles et al,
RHONE-POULENC AGROCHIMIE P.I.D. BP 9163
F-69263 Lyon Cedex 09(FR)

(54) **Préparation d'herbicides à groupe phosphonates et d'intermédiaires à partir de benzoxazines.**

(57) Préparation d'intermédiaires pour herbicides de formule

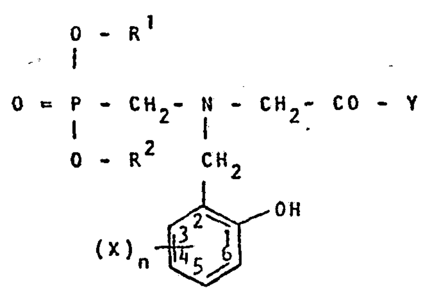

(I)

par réaction de phosphites organiques avec des benzoxazines de formule

$$Bzx - CH_2 - CO - Y \qquad (II)$$

dans lesquelles

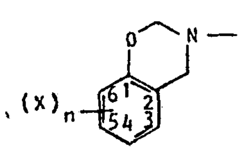

- X est un substituant habituel des phénols
- n est un entier de 0 à 4
- $R^1$ et $R^2$ sont H ou tels que $OR^1$ et $OR^2$ sont hydrolysables
- Y est OM ou $NR^3R^4$
- M est H ou est tel que COOM est un sel ou ester
- $R^3$ et $R^4$ sont H ou hydrocarbyle, l'une d'entre eux peuvent être $R^5-SO^2-$
- $R^5$ est un radical hydrocarboné éventuellement substitué.

Produits ainsi obtenus et leur utilisation.

EP 0 195 734 A2

Croydon Printing Company Ltd.

La présente invention concerne un nouveau procédé de préparation de produits intermédiaires pour la fabrication d'herbicides ainsi que certains de ces intermédiaires en tant que produits nouveaux.

Selon le procédé selon l'invention, on prépare un produit de formule

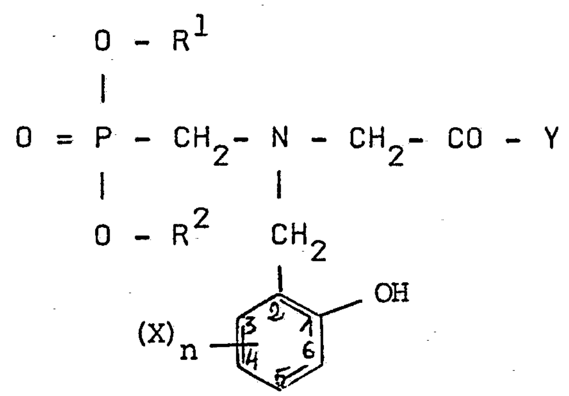

(I)

par réaction de phosphites de formule

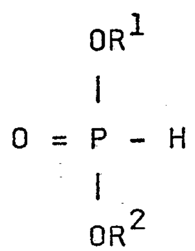

avec une benzoxazine de formule Bzx-$CH_2$-CO-Y (II) dans lesquelles

- Bzx représente le radical de formule :

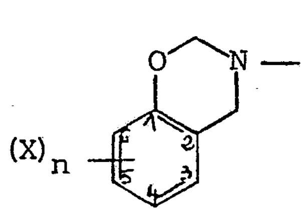

- X représente un substituant des phénols connu en soi, tel que par exemple
  . un atome d'halogène (notamment de fluor, chlore, brome ou iode) ou

. un radical hydrocarbyle (notamment alkyle, alkenyle, alkynyle, cycloalkyle, aryle) éventuellement substitué et ayant de préférence de 1 à 4 atomes de carbone,

étant entendu que les divers substituants X, s'il y en a plusieurs, peuvent être identiques ou différents et que, de préférence, au moins un substituant se trouve en position 4, un deuxième substituant, s'il y en a un, se trouvant de préférence en position 6,

- n est un nombre entier, positif ou nul, inférieur ou égal à 4, de préférence égal à 1 ou 2,
- $R^1$ et $R^2$, identiques ou différents, représentent l'atome d'hydrogène ou sont tels que $OR^1$ et $OR^2$ sont des groupements hydrolysables, $R^1$ et $R^2$ peuvent être notamment un radical alkyle ou aryle (de préférence alkyle ou phényle), éventuellement substitués, notamment par des substituants tels que ceux indiqués pour $R^5$ ci-après ; ils ont généralement, de 1 à 12 atomes de carbone et de préférence de 1 à 8 atomes de carbone,
- Y est un reste -OM ou $-NR^3R^4$,
- M est l'atome d'hydrogène ou un atome de métal alcalin ou d'ammonium ou un atome ou groupe tel que -COOM constitue une fonction sel ou ester (de préférence ester d'alcanol ayant de 1 à 4 atomes de carbone),
- $R^3$, $R^4$, identiques ou différents, représentent l'atome d'hydrogène ou un radical hydrocarbyle (notamment alkyle, alkenyle, alkynyle, cycloalkyle - spécialement cyclohexyle, ou aryle ou aralkyle), l'un d'entre eux pouvant être un groupe sulfonyle $R^5-SO_2-$ ($R^3$ et $R^4$ ont généralement de 1 à 18 atomes de carbone, de préférence de 1 à 4 atomes de carbone),
- $R^5$ représente un radical hydrocarboné, spécialement alkyle, aryle ou cycloalkyle, ces divers radicaux pouvant éventuellement être substitués ; comme substituants, on peut citer en particulier, les atomes

d'halogène et les groupes phényle, cyano, alkyle, alkoxyle, carboxylate d'alkyle, dans lesquels les groupes alkyle ont de préférence de 1 à 4 atomes de carbone ; $R^5$ a le plus souvent de 1 à 18 atomes de carbone, de préférence de 1 à 7 atomes de carbone, et plus spécialement de 3 à 7 atomes de carbone lorsqu'il s'agit d'un groupe cycloalkyle ; de préférence il s'agit d'un radical alkyle ayant de 1 à 4 atomes de carbone, éventuellement halogéné, notamment chloré ou fluoré, par exemple $CF_3$.

Les produits de formule (I) sont des produits nouveaux qui font partie de l'invention. Les produits de formule (II) font généralement partie de l'invention sauf lorsque l'une ou l'autre des conditions a, b, c, suivantes, prise isolément, est réalisée :

a) $R^3$ et $R^4$ sont l'atome d'hydrogène et que n est 1 et X est Br ou $CH_3$ ou méthoxy en position 4,

b) M est méthyle, n est 1 et X est méthoxy en position 4.

c) M est l'atome d'hydrogène, n est 1, et X est un tertiobutyle en position 4.

La réaction d'un phosphite avec un produit de formule (II) est effectuée soit en présence soit en absence de solvant, à température comprise entre 40° et 180°C, de préférence entre 70 et 130°C.

Comme solvants utilisables, on peut citer à titre non limitatif les hydrocarbures halogénés ou non, les éthers et esters (notamment les alcanoates d'alkyle, et plus particulièrement l'acétate d'éthyle), les nitriles.

La proportion des réactifs est généralement telle que le rapport molaire phosphite/composé de formule (II) soit compris entre 0,9 et 2, de préférence entre 1 et 1,5.

Les procédés de préparation de composés de formule (II) font également partie de l'invention.

Selon l'un de ces procédés, les produits de formule (II), dans laquelle X, n et Y ont la signification indiquée pour la formule (I) mais Y est de préference le radical -OM, se préparent commodément par réaction d'un phénol de formule

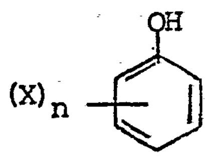

(III)

(dans laquelle X et n ont les significations déjà indiquées, et au moins une des positions sur le cycle benzénique en ortho du groupe hydroxyle est libre, c'est-à-dire non substituée)
avec du formaldéhyde et un dérivé d'aminoacide de formule

$NH_2-CH_2-CO-Y$ (IV)

(dans laquelle Y a la signification indiquée plus haut), la quantité molaire de formaldéhyde étant supérieure ou égale à 2 fois le nombre de moles du composé de formule (III) ou (IV) qui est en quantité molaire inférieure ou égal à l'autre. La température réactionnelle est généralement comprise entre 0° et 120°C, de préférence entre 10 et 80°C. On opère avantageusement en présence d'un agent basique; comme agent basique on peut utiliser une base organique ou de préférence minérale, par exemple un hydroxyde ou carbonate, alcalin ou alcalino-terreux et plus spécifiquement un dérivé de sodium ou de potassium; la quantité mise en oeuvre est généralement comprise entre 0,01 et 5 % en mole, de préférence entre 0,05 et 1 %, ces pourcentages étant comptés par rapport au phénol de formule (III) mis en oeuvre ; dans le cas où Y est un groupe OH, cette quantité doit être augmentée de la quantité d'agent basique nécessaire à neutraliser la fonction acide. Le milieu réactionnel est avantageusement alcoolique mais peut

aussi contenir d'autres solvants organiques ou même de l'eau, de préférence un solvant des réactifs de formule (III) et/ou (IV). Le formaldéhyde peut être mis en oeuvre sous forme de précurseur tel que le paraformaldéhyde.

Selon un autre procédé de préparation de composés de formule (II), notamment de composés dans la formule desquels Y est $-NR^3R^4$, et plus spécialement ceux dans lesquels Y est $-NR^3$ $(-SO_2-R^5)$, on prépare lesdits composés à partir de composés analogues de formule (II) dans laquelle Y est OM.

C'est ainsi que l'invention concerne un procédé de préparation de composé de formule :

$$Bzx - CH^2 - CO - \underset{\underset{R^{30}}{|}}{N} - SO_2 - R^5 \qquad (V)$$

dans laquelle Bzx, $R^5$ et n sont tels que définis pour les formules (I) et (II), et $R^{30}$ a la même signification que $R^3$ hormis l'atome d'hydrogène, ce procédé consistant à faire réagir un sulfonamide de formule :

$$R^{30} -NH- SO_2 - R^5 \qquad (VI)$$

avec un anhydride mixte de formule :

$$Bzx - CH_2 - CO - O- CO - O - alkyle \qquad (VII)$$

lui-même obtenu par réaction d'un produit de formule :

$$Bzx - CH_2 - COOH \qquad (VIII)$$

(de préférence sous forme salifiée) avec un chloroformiate d'alkyle (Cl - CO - O - alkyle ; alkyle a de préférence 1 à 4 atomes de carbone). Le produit de formule (VIII) est avantageusement utilisé sous forme de sel alcalin ou

d'ammonium. La réaction s'effectue avantageusement à température comprise entre -30 et +30°C en présence de solvant et, de préférence en présence de catalyseurs de transfert de phase tels que les sels d'acides forts (halogènures, sulfates, phosphates) d'ammonium quaternaire ; si on utilise un solvant dans lequel les sels formés en cours de réaction sont insolubles, il suffit alors de séparer le produit de réaction par filtration. C'est ainsi qu'on peut utiliser comme solvants des éthers et des esters, notamment le tétrahydrofuranne et l'acétate d'éthyle.

La réaction de l'anhydride mixte de formule (VII) avec le sulfonamide $R^5 - SO_2 - NH - R^{30}$ s'effectue avantageusement en milieu biphasique eau/solvant organique en présence d'un agent alcalin et d'un catalyseur de transfert de phase. La température est généralement comprise entre 0 et 50°C. Comme catalyseur de transfert de phase (utilisé généralement à raison de 0,1 à 10 % en poids par rapport à l'anhydride mixte) on peut citer les sels d'ammonium quaternaire et d'acide fort tels que les halogénures ou sulfates de tétraalkyl ammonium ou trialkyl aralkylammonium. Comme agent alcalin on utilise avantageusement un hydroxyde ou carbonate alcalin ou alcalino-terreux ou d'ammonium, de préférence un hydroxyde alcalin. Comme solvant organique on utilise un solvant organique non miscible à l'eau, par exemple $CH_2Cl_2$ ou les esters (notamment l'acétate d'éthyle).

La préparation de composés herbicides à partir de composés de formule (I) se fait habituellement par hydrogénolyse du groupement orthohydroxybenzyle de formule :

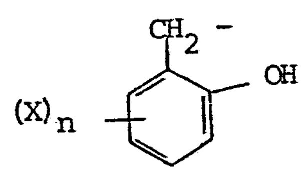

ce qui conduit à la formation de composés de formule :

$$
\begin{array}{l}
\qquad O-R^1 \\
\qquad | \\
O = P - CH_2 - NH - CH_2 - CO - Y \\
\qquad | \\
\qquad O-R^2
\end{array}
$$

lesquels peuvent soit être utilisés tels quels, soit être transformés en dérivés divers par des réactions telles que hydrolyse et/ou salification et/ou estérification et autres. Les composés herbicides qu'on peut ainsi obtenir sont, en général, des composés connus.

Cette réaction d'hydrogénolyse des composés de formule (I) s'effectue avantageusement en milieu aqueux ou alcoolique à température ambiante ou supérieure, à pression atmosphérique ou supérieure. Comme catalyseur on peut utiliser les catalyseurs habituels d'hydrogénolyse des radicaux benzyle ou benzyle substitués. Comme catalyseurs appropriés on peut citer le palladium, le platine, le nickel Raney. Ce catalyseur peut être utilisé avec ou sans support inerte. On peut aussi utiliser les métaux précités, spécialement le palladium et le platine sous forme de sels, d'hydroxydes, ou d'oxydes, lesquels se transforment en métal correspondant sous l'action de l'hydrogène. Comme catalyseur d'hydrogénolyse préféré, on utilise les catalyseurs à base de palladium comme le palladium sur charbon ou le palladium sur sulfate de baryum, ou l'hydroxyde de palladium sur charbon. En fin de réaction, on peut séparer le catalyseur par filtration et évaporer le filtrat ; on obtient ainsi un produit herbicide de bonne pureté (cette hydrogénolyse est connue en soi dans le cas d'un atome d'azote substitué par un groupe benzyle).

Lorsqu'on désire préparer des formes non estérifiées d'herbicides connues comme par exemple la N-phosphonométhyl glycine elle-même, on peut hydrolyser totalement ou partiellement le produit de formule (I) (lorsque $R^1$ et $R^2$ sont autres que l'atome d'hydrogène) de manière connue, par exemple par chauffage avec une solution aqueuse d'agent acide ou alcalin, notamment d'hydroxyde ou carbonate alcalin ou alcalino-terreux, ou d'acides chlorhydrique, bromhydrique, sulfurique, phosphorique, perchlorique, ou arylsulfonique. Cette hydrolyse peut aussi être accompagnée d'une salification ou d'une transformation d'autres dérivés herbicides.

Les exemples suivants, donnés à titre non limitatif illustrent l'invention et montrent comment elle peut être mise en pratique. Les produits chimiques ont été désignés selon la nomenclature française modifiée en plaçant, comme dans la nomenclature anglosaxonne, le chiffre désignant la position d'un substituant avant le nom de ce substituant.

Dans ces exemples, on désigne par B°z°x°, le radical de formule Bzx dans lequel n est égal à zéro.

Exemple 1 :

Dans un ballon de 50 $cm^3$ muni d'une agitation magnétique, d'un thermomètre, d'une ampoule de coulée, d'un réfrigérant et maintenu sous argon, on place 2,62 g (87,28 millimoles) de paraformaldéhyde, 3,5 ml de méthanol et 0,05 g d'hydroxyde de potassium solide. On solubilise à chaud puis refroidit. Une solution de glycinate d'éthyle (4,50 g ; 43,64 millimoles) dans le méthanol (1 $cm^3$) est alors coulée dans le milieu réactionnel à 20°C pendant 5 minutes. On ajoute alors le para-crésol (4,71 g ; 43,64 millimoles) puis chauffe au reflux pendant 20 minutes environ, période au bout de laquelle le méthanol est éliminé sous pression réduite à 0,2 bar et à une température voisine de 50°C.

Le résidu est chromatographié sur une colonne de silice (éluant hexane-acétate d'éthyle).

Après élimination des solvants sous pression réduite (0,2 bar) et séchage on obtient 3,65 g (15,5 millimoles) d'un produit huileux de formule

5-méthyl-B°z°x° - $CH_2$- $COOC_2H_5$

dont la structure est confirmée par spectroscopie (bande d'absorption infra-rouge à 1750 $cm^{-1}$).

Dans un ballon de 10 $cm^3$ on introduit 3,5 g (14,8 millimoles) de ce produit huileux et 2,07 g (14,8 millimoles) de phosphite de diéthyle. Le mélange est chauffé à une température voisine de 80°C pendant 55 minutes. On ajoute alors 0,20 g (1,48 millimole) de diéthylphosphite et chauffe au voisinage de 80°C pendant 35 minutes.L'excès de phosphite de diéthyle est éliminé sous pression réduite (0,27 bar) à une température voisine de 40°C.

Le résidu est constitué de phosphonate de formule :

$C_2H_5$ - O
|
O = P - $CH_2$- N -$CH_2$- CO - O - $C_2H_5$
|          |
$C_2H_5$ - O          $CH_2$- $C_6H_3$ (4-méthyl-1-hydroxy)

la numérotation des positions sur le radical phényle étant effectuée comme indiqué dans la formule (I).

Ce phosphonate (1,0 g ; 2,54 millimoles) est dissout dans 5,05 ml de soude méthanolique 1,01 N (5,05 millimoles). Le mélange est chauffé à une température voisine de 80°C pendant 3 heures. Le méthanol est éliminé sous pression réduite (0,2 bar) à une température voisine de 40°C. Le résidu est dissout dans 3 $cm^3$ d'eau distillée puis la phase aqueuse obtenue est lavée avec du chlorure de méthylène. La phase aqueuse est alors acidifiée à pH=2 avec HCl 6N puis extraite avec trois fois 30 $cm^3$ de chlorure de méthylène. Les extraits organiques réunis sont séchés

sur sulfate de magnésium. Après séparation par filtration et élimination du chlorure de méthylène sous pression réduite (0,2 bar) on obtient 550 mg (1,59 millimole) sous forme d'un solide blanc fondant à 102°C, de pureté voisine de 90 %, et ayant pour formule

$$\begin{array}{l} C_2H_5 - O \\ \qquad\quad | \\ \qquad O = P - CH_2 - N - CH_2 - CO - OH \\ \qquad\quad | \qquad\qquad | \\ C_2H_5 - O \qquad\quad CH_2 - C_6H_3 \text{ (4-méthyl-1-hydroxy)} \end{array}$$

la numérotation des positions sur le radical phényle étant effectuée comme indiqué dans la formule (I).

Ce produit est hydrogénolysable en produit de formule :

$$\begin{array}{l} C_2H_5 - O \\ \qquad\quad | \\ \qquad O = P - CH_2 - NH - CH_2 - COOH \\ \qquad\quad | \\ C_2H_5 - O \end{array}$$

selon les procédés connus.

<u>Exemple 2</u> :

Dans un ballon de 50 $cm^3$, on place 6,0 g (0,2 mole) de paraformaldéhyde, 8 $cm^3$ de méthanol et 0,1 g d'hydroxyde de potassium solide. On solubilise à chaud puis refroidit.

Une solution de glycinate de sodium (préparée à partir de glycine (7,50 g ; 0,1 mole), de soude en poudre (4,0 g ; 0,1 mole) et de méthanol (30 $cm^3$) est alors ajoutée goutte à goutte à la solution précédente à 20°C et sous agitation. On agite 20 minutes à 20°C. On ajoute alors 9,4 g (0,1 mole) de phénol et on chauffe à 60°C. Après 45 mn, on refroidit à 20°C et le précipité est filtré puis

lavé avec 3 fois 10 $cm^3$ d'éther éthylique et séché.

On obtient 8,47 g d'une poudre blanche qu'on purifie à 60ºC en l'agitant une heure dans 25 $cm^3$ de méthanol. Il y a alors dissolution presque complète. On laisse alors revenir la température à 20°C. Le précipité formé est filtré, lavé avec 3 fois 20 $cm^3$ d'éther éthylique puis séché sous pression réduite (0,27 bar ; 20°C).

On obtient ainsi 4,55 g (21,1 millimoles) de benzoxazine de pureté 90 % et de formule B°z°x°-$CH_2$-COONa.

Cette benzoxazine peut être traitée avec un phosphite comme indiqué à l'exemple 1 pour obtenir un produit de formule (I).

Exemple 3 :

Dans un ballon de 100 $cm^3$, on charge 6,0 g (0,2 mole) de paraformaldéhyde, 8 $cm^3$ de méthanol et une pastille de potasse. Le milieu est chauffé 15 mn à 45°C puis refroidi à 15°C. Une solution de glycinate de sodium (préparée à partir de glycine (7,50 g ; 0,1 mole), de soude pulvérisée (4,00 g ; 0,1 mole) et de méthanol (30 $cm^3$)) est alors ajoutée en 20 mn à la solution précédente sous agitation à 20°C. Le mélange obtenu est agité 30 minutes à 20°C.

On ajoute alors du para-crésol (10,81 g ; 0,1 mole) et on chauffe 35 mn à 50-60°C et on refroidit à 20°C. Le précipité est filtré puis lavé avec trois fois 25 $cm^3$ d'éther et séché.

On obtient ainsi 13,26 g (57,8 mmoles) de benzoxazine pratiquement pure (rendement : 57,8 %) et ayant pour formule 4-méthyl-B°z°x°-$CH_2$-COONa (bande d'absorption infra-rouge à 1600 $cm^{-1}$ et 1400 $cm^{-1}$).

Exemple 4 :

Dans un ballon de 500 $cm^3$ on charge du paraformaldéhyde (18,0 g ; 0,6 mole), du méthanol (24 $cm^3$), de l'hydroxyde de potassium solide (0,3 g) et on chauffe 15 mn à 45-50°C. On refroidit à 15°C et ajoute de la triéthylamine (4,2 $cm^3$ ; 3,03 g (30 millimoles)).

Une solution de glycinate de sodium (préparée à partir de 22,52 g = 0,3 mole de glycine) , de méthanol (90 $cm^3$) et d'hydroxyde de sodium solide (12,0 g ; 0,3 mole) est ajoutée goutte à goutte à la solution précédente à 15°C. Le milieu est ensuite agité 1 heure à 20°C. On ajoute alors du 2,4-dichlorophénol (48,9 g ; 0,3 mole) et on chauffe au reflux pendant 3 heures puis refroidit à 20°C.

Après filtration et traitement analogue à celui décrit à l'exemple 3 on obtient 42,0 g (0,148 mole) de benzoxazine (rendement 49,3 %) de formule 4,6-dichloro-B°z°x°-$CH_2$-COONa dont la structure est confirmée par spectroscopie.

Exemple 5 :

En opérant d'une manière analogue à celle décrite à l'exemple 4 mais à partir

- d'une solution de paraformaldéhyde (18,0 g ; 0,6 mole) et de potasse (0,3 g) dans le méthanol d'une part
- d'une solution de glycinate de sodium préparée à partir de glycine (22,52 g ; 0,3 mole), d'hydroxyde de sodium (12,0 g ; 0,3 mole) dans le méthanol (90 $cm^3$) d'autre part
- de 38,57 g (0,300 mole) de parachlorophénol,

on obtient 24,5 g (98,3 millimoles) de benzoxazine pure (rendement 32,7 %) de formule 4-chloro-B°z°x°-$CH_2$-COONa. Les benzoxazines des exemples 3 à 5 peuvent être traitées avec des phosphites comme indiqué aux exemples 1 et 2.

Exemple 6 :

Dans un ballon de 20 $cm^3$, on place :

-1,15 g (5 millimoles) de benzoxazine préparée à l'exemple 3,

-5 $cm^3$ d'acétate d'éthyle sec,

-0,11 g de chlorure de tétra(n-butyl) ammonium.

La suspension obtenue est refroidie à 10°C. On ajoute du chloroformiate d'éthyle (0,48 $cm^3$ ; 5 millimoles) à -10°C puis on laisse remonter la température à 20°C. On agite pendant 2 heures puis refroidit à 10°C. Il s'est formé le produit de formule 4-méthyl-B°z°x°-$CH_2$-CO-O-CO-O-$C_2H_5$.

On ajoute alors du N-méthyl méthane sulfonamide (0,59 g ; 5,5 millimoles) et coule de la soude aqueuse à 50% (poids/poids) (0,44 g ; 5,5 millimoles), goutte à goutte et en maintenant la température à + 10°C. Après 25 mn le milieu est dilué avec 5 $cm^3$ d'eau. La phase organique est recueillie et la phase aqueuse est extraite avec 4 fois 10 $cm^3$ d'acétate d'éthyle. Les extraits organiques réunis sont lavés avec 10 $cm^3$ d'eau distillée puis séchés sur sulfate de sodium. Après séparation par filtration et concentration sous pression réduite (0,2 bar) à 30°C on obtient 1,25 g d'un résidu huileux qui cristallise lentement vers 4°C. On purifie par recristallisation dans un mélange d'acétate d'éthyle et de cyclohexane et obtient un produit fondant à 134°C et ayant pour formule

4-méthyl-B°z°x°-$CH_2$-CO-N($CH_3$)-$SO_2$-$CH_3$

Dans un ballon de 10 $cm^3$ on introduit 0,317 g (1,062 millimole) de benzoxazine ainsi préparée et 0,147 g (1,065 millimoles) de phosphite de diéthyle ainsi que 1 $cm^3$ de toluène.

Le mélange est chauffé à 80°C pendant 2 heures, puis au reflux pendant 5 heures, puis on élimine le toluène sous pression réduite (0,2 bar) à 30°C.

Le résidu (0,450 g) est chromatographié sur silice (10 g) en éluant avec un mélange chlorure de méthylène/méthanol. Après élimination des solvants sous

pression réduite (0,2 bar) à 30°C et séchage sous vide poussé (27 millibars ; 20°C) ; on obtient un solide blanc fondant à 79°C (rendement 41,6 %) et ayant pour formule :

```
C2H5 - O
       |
  O =  P - CH2- N -CH2- CO - N(CH3)-SO2-CH3
       |        |
C2H5 - O        CH2 -C6H3 (4-méthyl-1-hydroxy)
```

la numérotation des positions sur le radical phényle étant effectuée comme indiqué dans la formule (I).

Ce produit peut être hydrogénolysé comme indiqué à l'exemple 1.

## REVENDICATIONS

1) Procédé de préparation de produit de formule

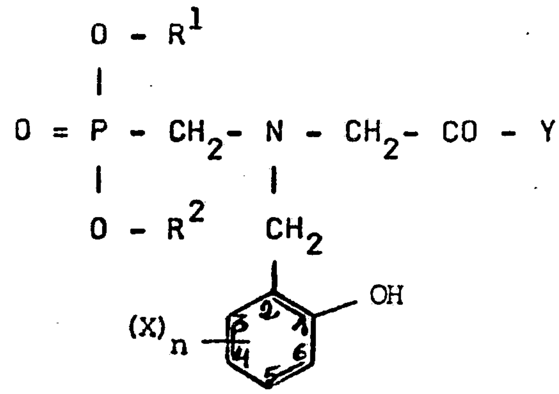

(I)

caractérisé en ce qu'on fait réagir un phosphite de formule

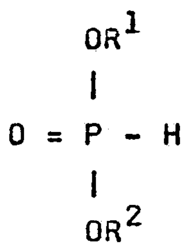

avec une benzoxazine de formule $Bzx-CH_2-CO-Y$ (II) dans lesquelles

- Bzx représente le radical de formule :

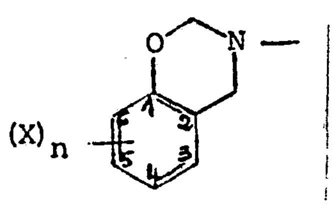

- X représente un substituant des phénols connu en soi, tel que par exemple
  . un atome d'halogène (notamment de fluor, chlore, brome ou iode) ou

. un radical hydrocarbyle (notamment alkyle, alkenyle, alkynyle, cycloalkyle, aryle) éventuellement substitué.

- n est un nombre entier, positif ou nul, inférieur ou égal à 4, de préférence égal à 1 ou 2,
- $R^1$ et $R^2$ représentent l'atome d'hydrogène ou sont tels que $OR^1$ et $OR^2$ sont des groupements hydrolysables,
- Y est un reste -OM ou $-NR^3R^4$,
- M est l'atome d'hydrogène ou un atome de métal alcalin ou d'ammonium ou un atome ou groupe tel que -COOM constitue une fonction sel ou ester,
- $R^3$, $R^4$, identiques ou différents, représentent l'atome d'hydrogène ou un radical hydrocarbyle (notamment alkyle, alkenyle, alkynyle, cycloalkyle spécialement cyclohexyle, ou aryle ou aralkyle), l'un d'entre eux pouvant être un groupe sulfonyle $R^5-SO_2-$,
- $R^5$ représente un radical hydrocarboné éventuellement substitué, spécialement un radical alkyle, aryle ou cycloalkyle.

2) Procédé selon la revendication 1 caractérisé en ce que la réaction est effectuée entre 40 et 180°C, de préférence entre 70 et 130°C, éventuellement en présence d'un solvant.

3) Produits nouveaux, utilisables notamment pour la fabrication de produits herbicides, caractérisés en ce qu'ils ont pour formule

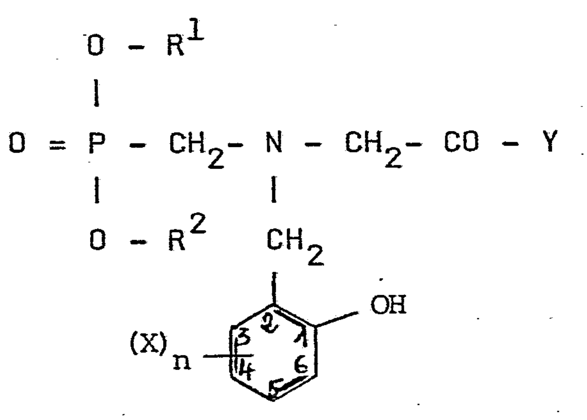

(I)

dans laquelle :

- X représente un substituant habituel des phénols, tel que par exemple
  . un atome d'halogène (notamment de fluor, chlore, brome ou iode) ou
  . un radical hydrocarbyle (notamment alkyle, alkenyle, alkynyle, cycloalkyle, aryle) éventuellement substitué.
- n est un nombre entier, positif ou nul, inférieur ou égal à 4, de préférence égal à 1 ou 2,
- $R^1$ et $R^2$ représentent l'atome d'hydrogène ou sont tels que $OR^1$ et $OR^2$ sont des groupements hydrolysables,
- Y est un reste -OM ou $-NR^3R^4$,
- M est l'atome d'hydrogène ou un atome de métal alcalin ou un groupe ammonium ou un atome ou groupe tel que -COOM constitue une fonction sel ou ester,
- $R^3$, $R^4$, identiques ou différents, représentent l'atome d'hydrogène ou un radical hydrocarbyle (notamment alkyle, alkenyle, alkynyle, cycloalkyle spécialement cyclohexyle, ou aryle ou aralkyle), l'un d'entre eux pouvant être un groupe sulfonyle $R^5-SO_2-$,
- $R^5$ représente un radical hydrocarboné éventuellement substitué, spécialement un radical alkyle, aryle ou cycloalkyle.

4) Produits selon la revendication 3 caractérisés en ce qur $R^1$ et $R^2$ sont des radicaux alkyle ou aryle éventuellement substitués,

5) Produits selon la revendication 4 caractérisés en ce que $R^1$ et $R^2$ ont de 1 à 12 atomes de carbone, de préférence de 1 à 8 atomes de carbone.

6) Produits selon l'une des revendications 3 à 5 caractérisés en ce que $R^1$ et/ou $R^2$ et/ou $R^5$ sont substitués par des atomes d'halogène ou des groupes phényle, cyano, alkyle, alkoxyle, carboxylate

7) Produits selon l'une des revendications 3 à 6 caractérisés en ce que $R^5$ à de 1 à 18 atomes de carbone, de préférence de 1 à 7 atomes de carbone.

8) Produits utilisables notamment dans le procédé selon la revendication 1, caractérisés en ce qu'ils ont pour formule

Bzx - $CH_2$-CO - Y

dans laquelle

- Bzx représente le radical de formule :

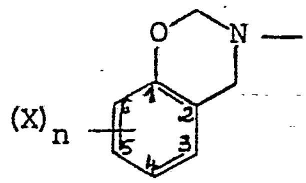

- X représente un substituant habituel des phénols, tel que par exemple
  . un atome d'halogène (notamment de fluor, chlore, brome ou iode) ou
  . un radical hydrocarbyle (notamment alkyle, alkenyle, alkynyle, cycloalkyle, aryle) éventuellement substitué.
- n est un nombre entier, positif ou nul, inférieur ou égal à 4, de préférence égal à 1 ou 2,
- Y est un reste -OM ou $-NR^3R^4$,
- M est l'atome d'hydrogène ou un atome de métal alcalin ou un groupe ammonium ou un atome ou groupe tel que -COOM constitue une fonction sel ou ester,
- $R^3$, $R^4$, identiques ou différents, représentent l'atome d'hydrogène ou un radical hydrocarbyle (notamment alkyle, alkenyle, alkynyle, cycloalkyle spécialement cyclohexyle, ou aryle ou aralkyle), l'un d'entre eux pouvant être un groupe sulfonyle $R^5-SO_2-$,
- $R^5$ représente un radical hydrocarboné éventuellement substitué, spécialement un radical alkyle, aryle ou cycloalkyle,

sous réserve que l'une ou l'autre des conditions suivantes ne soit pas réalisée

a) $R^3$ et $R^4$ sont l'atome d'hydrogène et n est 1 et X est Br ou $CH_3$ ou méthoxy en position 4,

b) M est méthyle, n est 1 et X est méthoxy en position 4.

c) M est l'atome d'hydrogène, n est 1, et X est un tertiobutyle en position 4.

9) Produits selon la revendication 8 caractérisés en ce que les substituants vérifient l'une et/ou l'autre des conditions définies dans l'une des revendications 4 à 7

10) Procédé de préparation de produits tels que définis dans l'une des revendications 8 ou 9, caractérisé en ce qu'on fait réagir un phénol de formule

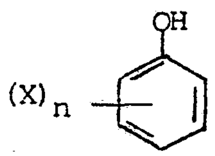

(III)

dans laquelle X et n ont les significations indiquées dans l'une des revendications 3 à 7, avec du formaldéhyde (ou un des ses précurseurs) et un dérivé d'aminoacide de formule

$NH_2-CH_2-CO - Y$ (IV)

(dans laquelle Y est OM, M ayant la signification indiquée dans ces revendications) et la quantité molaire de formaldéhyde étant supérieure ou égale à 2 fois le nombre de moles du composé de formule (III) ou (IV) qui est en quantité molaire inférieure ou égal à l'autre.

11) Procédé selon la revendication 10 caractérisé en ce que la température est comprise entre 0 et 120°C, de préférence entre 10 et 80°C.

12) Procédé de préparation de composé de formule :

$$Bzx - CH_2 - CO - \underset{\displaystyle R^{30}}{\underset{|}{N}} - SO_2 - R^5$$

dans laquelle $R^{30}$ a la même signification que $R^3$ hormis l'atome d'hydrogène et $R^3$, Bzx, $R^5$ et n sont tels que définis dans l'une des revendications 1 à 9, ce procédé consistant à faire réagir un sulfonamide de formule :

$$R^{30} - NH - SO_2 - R^5 \qquad (VI)$$

avec un anhydride mixte de formule :

$$Bzx - CH_2 - CO - O - CO - O - alkyle \qquad (VII)$$

13) Procédé selon la revendication 12 caractérisé en ce que la réaction est effectuée entre 0 et + 50°C en présence d'un solvant.

14) Produits nouveaux de formule

$$Bzx - CH_2 - CO - O - CO - O - alkyle \qquad (VII)$$

dans laquelle
Bzx à l'une des significations indiquées dans l'une des revendications 1 à 9, alkyle étant un groupe alkyle ayant de préférence de 1 à 4 atomes de carbone.

15) Procédé de préparation de produits tels que définis dans la revendication 14 caractérisés en ce qu'on fait réagir un produit de formule Bzx- $CH_2$- COOH éventuellement salifié avec un chloroformiate d'alkyle, Bzx ayant la signification indiquée à la revendication 14.

16) Procédé de préparation de composés, utilisables notamment comme herbicides ou pour la préparation d'herbicides, caractérisé en ce qu'on hydrogénolyse un produit selon l'une des revendications 3 à 7, cette hydrogénolyse étant éventuellement accompagnée d'une hydrolyse et/ou salification.